# EUROPEAN PATENT SPECIFICATION

(11) **EP 3 782 565 B1**
(45) Date of publication and mention of the grant of the patent: **07.05.2025**
(21) Application number: 20188689.2
(22) Date of filing: 30.07.2020
(51) Int. Cl.: A61B 17/70, A61B 17/72, A61B 17/88

(54) **VERTEBRAL FIXATION DEVICE**
WIRBELFIXIERUNGSVORRICHTUNG
DISPOSITIF DE FIXATION VERTÉBRALE

(30) Priority: 31.07.2019 TW 108127151
(43) Date of publication of application: 24.02.2021
(73) Proprietor: Apcore Medical Technology Co., Ltd., Taicang City (CN)
(72) Inventor: Lu, Shih-Chun, 114 Taipei City (TW); Lin, Shih Hung, 114 Taipei City (TW); Lin, Ying-Lien, 114 Taipei City (TW); Luo, Guan-Shin, 114 Taipei City (TW); Ting, Po-Yao, 114 Taipei City (TW)
(74) Representative: Aseglio-Gianinet, Romina

(56) References cited:
- EP-A1- 2 921 142
- US-A1- 2009 182 336

## Description

### BACKGROUND OF THE INVENTION

### 1. Field of the Invention

The present invention relates to a vertebral fixation device, and in particular, to a vertebral fixation device having an expanding device and a porous device.

### 2. The Prior Arts

In the operation of filling or inserting the medical filler into the bone, the current surgical methods commonly include the following:

When using a mechanical expanding device (such as patents US20110196494, US20110184447, US20100076426, US20070067034, US20060009689, US20050143827, US20220052623, US6354995, US6676665) for bone expanding to generate a space in the bone, the expanding device is taken out after expanding, then a covering device is inserted, and then the operation for filling or stuffing of the medical filler is performed. This type of operation has the following disadvantages: Mechanical expanding devices crush the cancellous bone when expanding and the crushed fragments often fall into the mechanical expanding device, which causes the mechanical expanding device to get stuck, and makes the expanded element unable to be retracted (recovered to a contracted state), this results in that the entire mechanical expanding device is stuck. Thereby, it cannot be retracted from the expanded position.

When a filling expanding device (such as patents US5972015, US6066154, US6235043, US6423083, US6607544, US6623505, US6663647, US6716216) for bone expanding to generate a space in the bone are used, the expanding device is taken out after expanding, then a covering device is inserted, and then the operation for filling or stuffing of the medical filler is performed. Because the filling expanding device mostly puts a balloon into the bone, and uses high pressure to inject liquid (such as water) into the balloon (a variety of balloons are used for different needs), the balloon is inflated to push the cancellous bone into the bone to achieve the purpose of expanding. However, the device or method has many disadvantages, for example, the balloon must be connected to a nozzle, so when the filling of the liquid is under high pressure, it may cause the balloon to fall off from the nozzle, and the balloon may even burst.

Without expanding in advance, the covering device is directly placed into the bone and the medical filler is injected, and the pressure under which the medical filler is injected into the covering device is used to achieve the effect of spreading the bone. Under such a situation, the covering device is both an expanding device and a vertebrae fixation device when infusing the medical filler (such as patents TWI321467, TW201112995, US6248110). Alternatively, the covering device is even abandoned, and an infusion device is directly used to inject the medical filler into the surgical position to enhance the fixation of the surgical position (such as patent US5514137). This kind of surgery has the following disadvantages: Because no expanding is performed first, the range of infusion cannot be accurately controlled, so that the direction after finishing infusion may be different from that originally expected by the doctor, and it is even found that the covering device does not completely support the bone or the medical filler flows around in the bone after the medical filler is injected, and the medical filler may even flow out of the bone, or the concentration of the slurry medical filler may be too thin or the particle is too small, which makes it difficult to support the bone when infusing the medical filler, and greatly reduces the original effect.

The mechanical expanding device can be used as a vertebrae fixation device (such as patents US20120071977, US20110046739, US20100069913, US20100217335, US20090234398, US20090005821). After the mechanical expanding device is implanted into the bone and the bone is opened, the medical filler is injected, the medical filler is allowed to cover the mechanical expanding device, and the mechanical expanding device and the medical filler are left in the human body together after the filling is finished. This kind of surgical method uses no covering device, the flow direction of the medical filler cannot be effectively controlled, and thus it is possible for the medical filler to flow around the bone, and even to flow out of the bone. In addition, the medical filler cannot effectively and completely cover the mechanical expanding device, and the mechanical expanding device may thus slowly contract from a fully expanded state to an incompletely expanded state. As a result, the bones are not completely expanded, which means that the original purpose of the vertebrae fixation device is lost.

EP2921142A1 discloses a device for bone fixation that includes: an expanding unit and an enclosing unit. The expanding unit includes two or more expanding structures capable of switching between an expanded state and a contracted state. The expanding unit includes a first expanding structure located near a first end thereof and a second expanding structure located near a second end thereof. The enclosing unit encloses the expanding unit and has a first end and a second end. The first end of the enclosing unit is secured at the first end of the expanding unit, and the second end of the enclosing unit is secured at the second end of the expanding unit. When the device for bone fixation is placed inside a bone, the expanding structures of the expanding unit are switched to the expanding state and thereby propping up the enclosing unit.

US20090182336A1 discloses an apparatus and methods for bone fracture repair. The apparatus may include a structural support for positioning a first bone segment relative to a second bone segment. The apparatus may include an anchoring substrate. The anchoring substrate may be configured to compress the first bone segment to the second bone segment. The anchoring substrate may transmit tension from a distal bone segment anchor in the first bone segment to a proximal bone segment anchor in the second bone segment. The apparatus may be configured to be deployed percutaneously in an inner cavity of a bone. The apparatus may be installed in an open fracture. The apparatus may be expanded, self-expanding or configured for mechanical actuation. Some embodiments of the apparatus may include a central axis member that may be used in conjunction with expansion of one or both of the structural support and the anchoring substrate to configure the apparatus.

A covering device can be used to cover the expansion device as a vertebral fixation device (such as patent US 10080595). The covering device can effectively prevent fragments of the crushed cancellous bone from falling into the expansion device during expanding, so that the expansion device can be repeatedly expanded and contracted in the bone for expanding to adjust the direction of expanding or the size of the expanding range, and to control the range of infusion of the medical filler by the covering device when infusing the medical filler. After the infusion is finished, the medical filler can completely cover the expansion device. However, the pores of the covering device of this invention are not three-dimensional connecting pores, so the effect of the perfused medical filler and bones to achieve interdigitate is poor. Secondly, the medical filler in the covering device is solid, and its strength is far higher than that of cancellous bone in the bone, which is likely to cause stress concentration, which makes it difficult for bone cells near the implant to grow after surgery.

The present invention uses a three-dimensional porous device to control the infusion range of the medical filler, and since the three-dimensional porous device has a three-dimensional connecting pore structure, the medical filler subsequently injected is closer to the structure of the cancellous bone and less likely to have stress concentration. In addition, the medical filler flows through the three-dimensional connecting pore structure and contacts the vertebrae, which is more likely to achieve an interdigitate effect. Furthermore, the three-dimensional porous device can apply biodegradable materials, and the medical filler will form countless three-dimensional connecting channels therein with the three-dimensional porous device degraded later in the body, and the bone cells can grow into the medical filler through these connecting channels and form a denser connection with the medical filler.

### SUMMARY OF THE INVENTION

The present invention is defined in claim 1 while preferred embodiments are set forth in the dependent claims.Associated surgical methods are also described herein to aid understanding the invention. These methods do not form part of the invention.

The present invention uses an adjustable expanding device to repeatedly expand and contract in the vertebrae to expand a space, and thereby adjust the scope and size of the space. After the expanding device expands a space, the pre-compressed porous device in the expanding device will expand and fill this space, and then a medical filler is injected into the porous device, so that the medical filler is interdigitated into the vertebrae and connected to the vertebrae by the porous device. The porous device can effectively control flow direction and a infusion range of the medical filler, and prevent the medical filler from scrambling in the vertebrae. On the other hand, because the porous device is a slurry structure with three-dimensional connecting pores, the injected medical filler will be closer to the structure of cancellous bone in the vertebrae, and mechanical performance thereof is also closer to cancellous bone, so the stress concentration problem is decreased, and postoperative bone cell growth is facilitated. Furthermore, the structure that forms the porous device will slowly degrade in the body and will form multiple connecting channels in the medical filler for the growing of bone cells, and more close combination to the medical filler.

An object of the present invention is to provide a vertebral fixation device.

The other object of the present invention is to provide a vertebral fixation device having an expanding device.

Another object of the present invention is to provide a vertebral fixation device having a porous device.

Yet another object of the present invention is to provide a vertebral fixation device having an expanding device and a porous device, the porous device being disposed inside the expanding device.

Still another object of the present invention is to provide a vertebral fixation device with a function of expanding vertebrae.

Another object of the present invention is to provide an expanding device which can repeatedly expand and contract a vertebral fixation device.

Still another object of the present invention is to provide a porous device that can control the flow direction and an infusion range of a medical filler.

Another object of the present invention is to provide a vertebral fixation device having a compressible and expandable porous device.

The other object of the present invention is to provide a vertebral fixation device that can be filled with a medical filler through a hollow operating lever.

Another object of the present invention is to provide a vertebral fixation device that can detach the expanding device from the operating lever and leave the expanding device in the vertebrae.

Still another object of the present invention is to provide a vertebral fixation device that can maintain an expanding device in an expanded state by a fixing mechanism.

A vertebral fixation device of the present invention comprises: an expanding device having a fixing end and a top end, wherein the expanding device is configured to be adjusted between an expanded state and a contracted state; and a three-dimensional porous device having a front end and a connecting end, wherein the front end of the three-dimensional porous device is connected with the top end of the expanding device, and the connecting end of the three-dimensional porous device is adapted for fixing to the fixing end of the expanding device; characterized in that the three-dimensional porous device is disposed inside the expanding device, and in that the expanding device and the three-dimensional porous device are configured to be placed into the vertebrae, the expanding device is configured to be adjusted to an expanded state to expand a space, and the three-dimensional porous device inside the expanding device is configured to expand and fill into the space, a medical filler is fillable into the three-dimensional porous device from the fixing end of the expanding device, and in that the three-dimensional porous device is configured effectively to control a flow direction and infusion range of the medical filler.

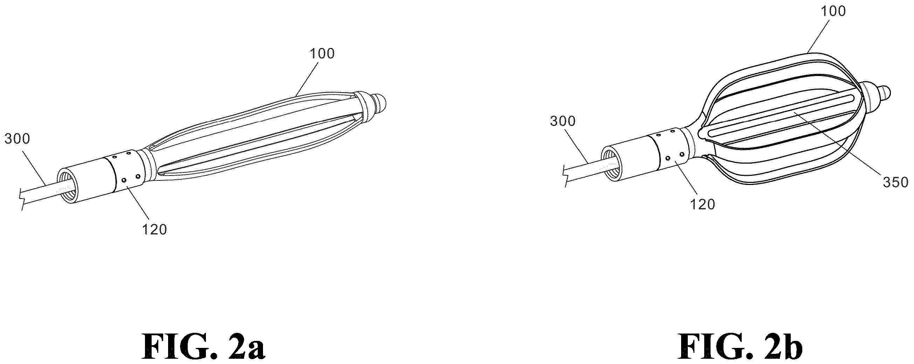

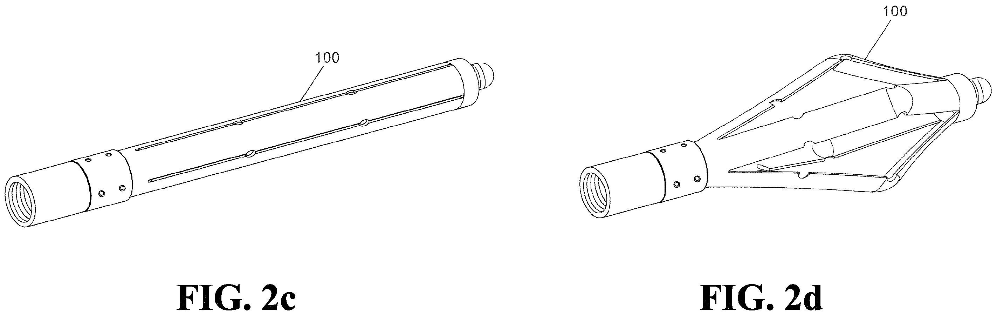

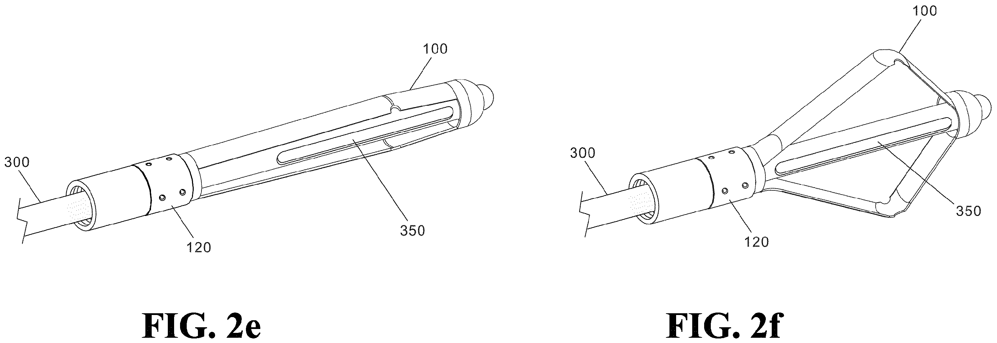

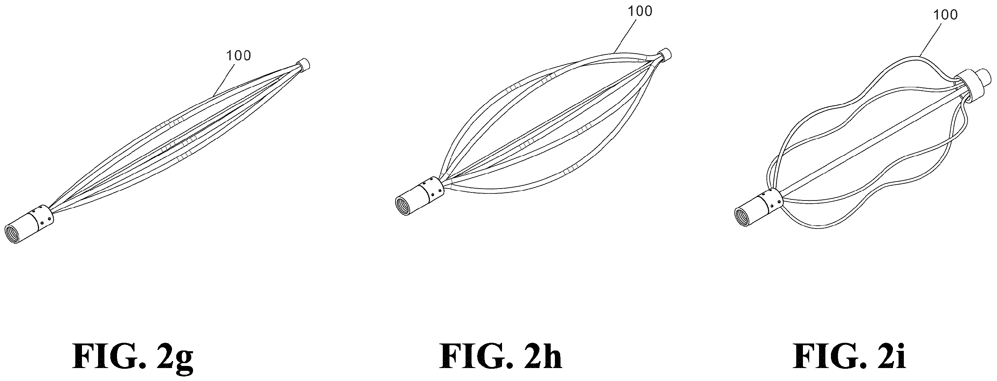

The above expanding device may be any conventional expanding device, such as patents US20110196494, US20110184447, US20100076426, US20070067034, US20060009689, US20050143827, US20220052623, US6549995, US6676665, US20120071977, US20110046739, US20100069913, US20100217335, US20090234398, US20090005821, etc., or the expanding devices described in patents such as TW585091 and TWI318110, the expanding device uses mechanical force to open a space, rather than using the pressure of a medical filler to open the space, so that the concentration of the medical filler can be avoided from being too dilute to expand the space, or from being too thick resulting in poor infusion or even excessive infusion pressure that causes the injection tool to burst. The expanding device which is lantern-shaped (as shown in FIGs. 2a, 2b, 2c, 2d, 2e, 2f, 2g, 2h, 2i) is preferred.

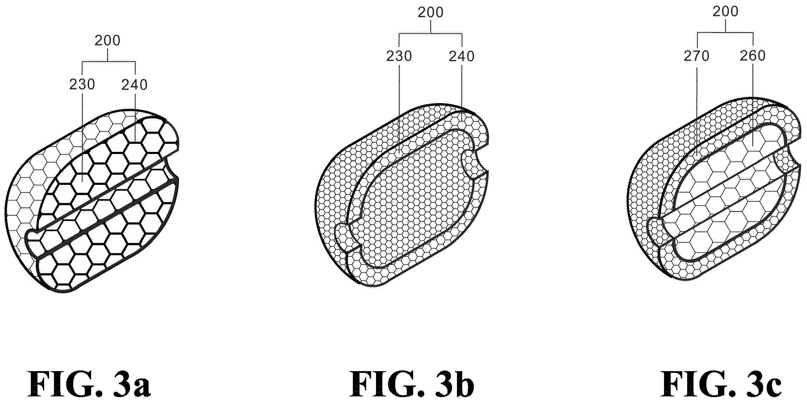

The structure of the three-dimensional porous device may be a three-dimensional connecting pore structure (as shown in FIGs. 3a, 3c), or may at least partly be a three-dimensional connecting pore structure (as shown in FIG. 3b). The three-dimensional porous device can even prevent the medical filler flowing out of the vertebrae, causing a risk to the patient, and effectively achieve the interdigitate effect, so that the medical filler and the vertebrae are connected more closely. In addition, the size, porosity, etc. of the three-dimensional connecting pore structure can be adjusted to not only control the overall mechanical strength of the medical filler and the three-dimensional porous device to make its mechanical performance closer to the strength of cancellous bone in the vertebrae for preventing stress concentration and being beneficial to the growth of bone cells after surgery, but also be used to adjust the smoothness of the infusion of the medical filler into the three-dimensional porous device.

The aforementioned three-dimensional connecting pore structure may further be a three-dimensional connecting pore structure with a fixed pore size (see FIG. 3a), a three-dimensional connecting pore structure with different pore sizes (see FIG. 3c), or a three-dimensional connecting pore structure being hollow inside (see Figure 3b) can be selected with three-dimensional porous devices with appropriate pore sizes according to the different thicknesses of the medical filler or bone looseness. On the one hand, the smoothness of the infusion of the medical filler can be increased. On the other hand, the mechanical performance of the completely formed medical filler and the three-dimensional porous device can be adjusted through the selection of the pore size.

The porous device is an expandable porous device, and the expandable porous device is pre-compressed and placed in the expanding device, and after the expanding device is expanded to create a space, the space may be automatically filled to facilitate subsequent infusion of the medical filler. The expandable porous device can be foam, sponge, or any compressible/expandible porous elastomer.

In the above three-dimensional porous device, the material forming the structure of the porous device may be any conventional biocompatible material, such as polyethylene, polyurethane, polyvinyl alcohol, nylon, silicone, etc., or further be a biodegradable material such as polylactic acid, gelatin, alginate, polyglycolic acid, polyhydroxy fatty acid ester, polychinolactone, etc. In this way, after the porous device and the medical filler are implanted in the human body, the porous device can slowly degrade in the human body, the structure of the porous device will form multiple connecting channels in the medical filler, and the bone cells can grow into the medical filler through these connecting channels, forming a closer intertwined connection with the medical filler. The material of the porous device is preferred to be a biodegradable material.

In the structure mentioned above forming a three-dimensional porous device, the curved plane boundary of any pore is composed of the above-mentioned material in shape of filament/thin line, and the diameter of the filament/thin line (hereinafter refer to as the structure diameter) may be smaller than 3000µm, the diameter of the connecting channel left from the porous device after degradation in the body can be controlled through the selection of the structure diameter. The structure diameter of the porous device is preferred to be 10µm to 1000µm. It is also easier for bone cells to grow in a range of 100µm-500µm.

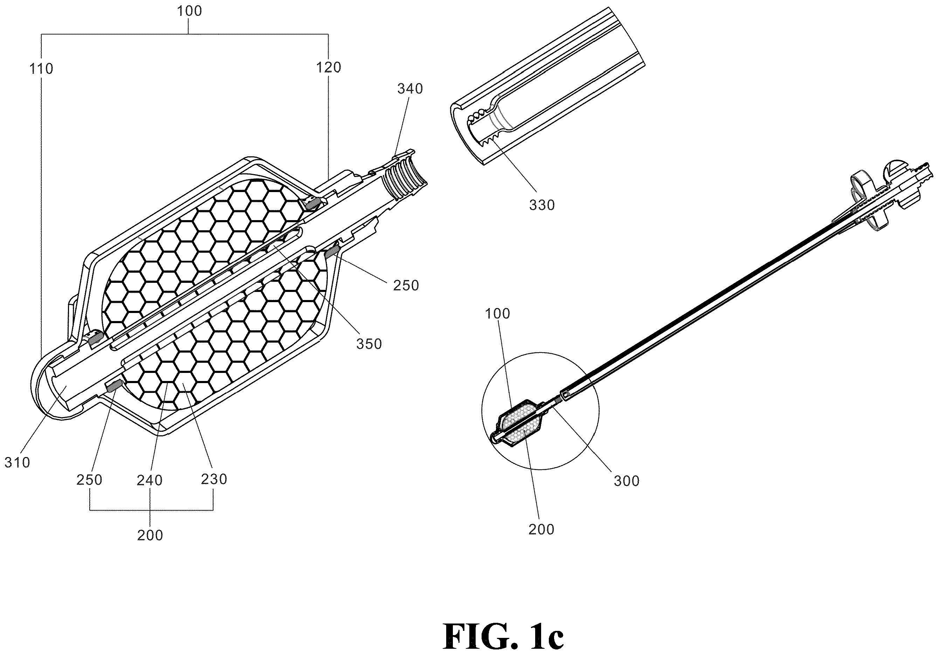

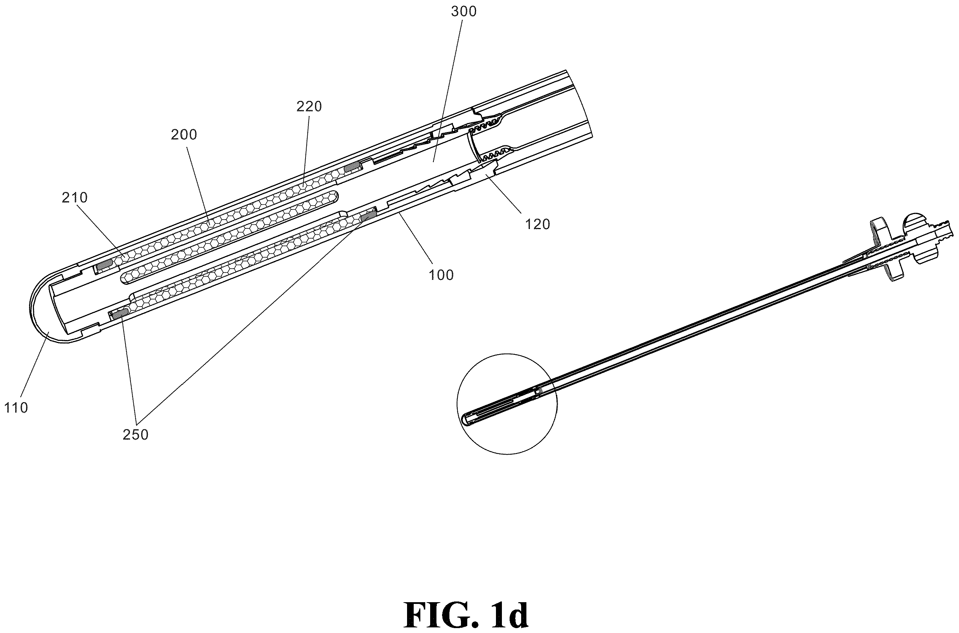

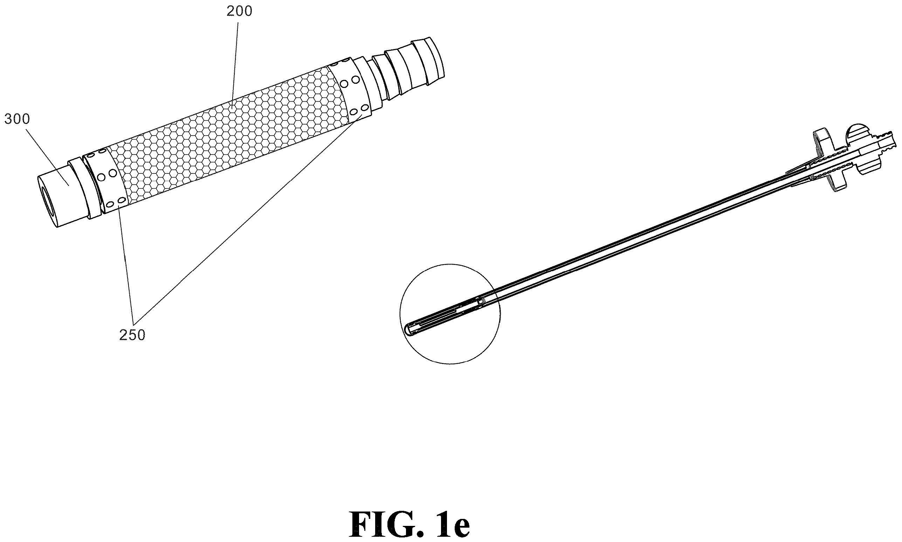

In the above three-dimensional porous device, the front end, the connecting end of the porous device can be respectively fixed to the top end/the fixing end of the expanding device by ring-shaped fixing members (as shown in FIGs. 1c, 1d, 1e, with numeral 250) or any conventional fixing manner. In this way, the porous device can be prevented from being displaced due to the excessive infusion pressure of the medical filler, which would cause the medical filler to overflow in an unexpected filling direction, and cause the patient possible paralysis or death.

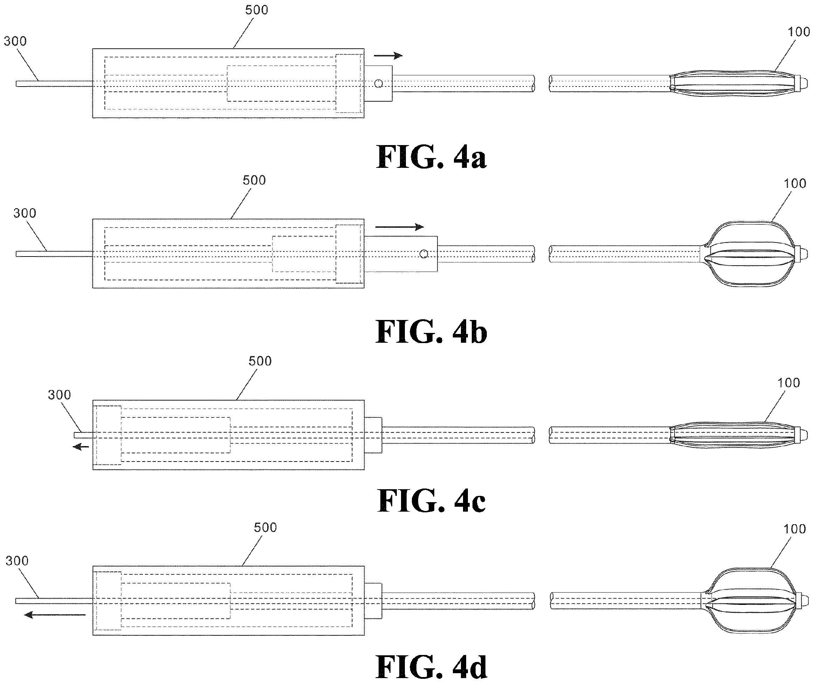

The vertebral fixation device may further include an operating lever with a connecting end and a manipulating end, wherein the connecting end is connected to the top end or the fixing end of the expanding device. The expanding device is adjusted to be in an expanded state or contracted state by relative movement of stretching or rotating (as shown in FIGs. 4a-4d) with the operating lever (as shown in FIGs. 4a-4d, with numeral 300).

The operating lever mentioned above may further be a hollow operating lever, and the hollow operating lever inside the expanding device has one or more perforations (for example, as shown in FIG. 1c, with numeral 350) allowing the medical filler to flow to the porous device through the perforations. The perforation can be any known form of perforation, such as grooves, pores, etc.

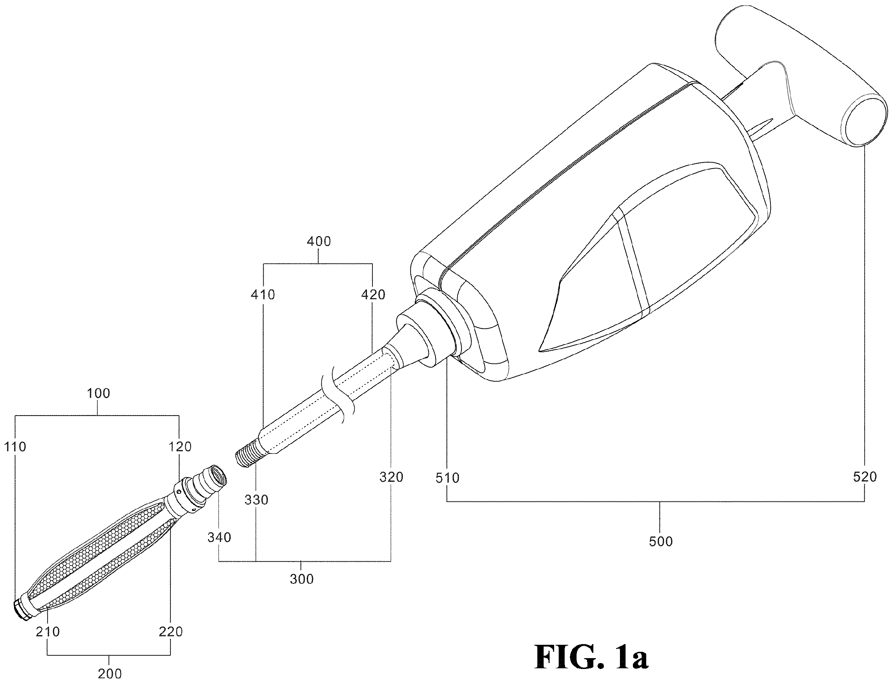

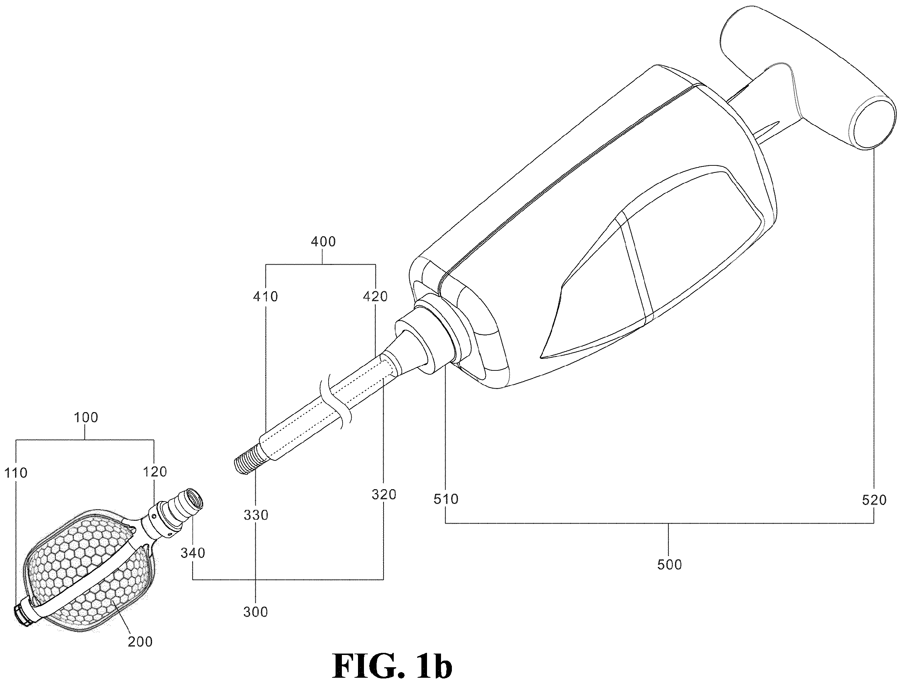

The operating lever may further include a second detachable mechanism (for example, as shown in FIGs. 1a, 1b, 1c, with numeral 330). After the medical filler is filled in the expanding device and the porous device, the second detachable mechanism detaches the expanding device from at least part of the operating lever, and leaves the expanding device and the porous device in the vertebrae. The second detachable mechanism may be any conventional detachable mechanism, such as screwing, engaging, locking or buckling, while screwing is preferred.

The above operating lever may further include a fixing mechanism (for example, FIGs. 1a, 1b, 1c, with numeral 340). After the expanding device is expanded to be in the expanded state, the expanding device may be maintained in the expanded state by the fixing mechanism to facilitate the subsequent expansion of the porous device and fill the expanding device, and the medical filler is filled into the expanding device. The fixing mechanism may be any conventional fixing mechanism, such as an expanding and fixing mechanism, a buckling fixing mechanism, a locking fixing mechanism, or a screwing fixing mechanism, etc., while the engaging and fixing mechanisms are preferred.

The above-mentioned medical filler may be any conventional consolidable and slurry medical filler, such as bone cement. The above-mentioned consolidable and slurry medical filler preferably is a medical filler with osteo-conductive and/or osteo-inductive materials added thereto, such as the conventional hydroxyapatite, calcium phosphate-based bone fillers, while it is preferred to add bone-leading medical fillers, such as the conventional SrHA-based medical filler.

A vertebral fixation device of the present invention can include an operating lever having a connecting end and a manipulating end, wherein the connecting end is configured for connection to the top end of the expanding device, and wherein the expanding device is configured to be adjusted between an expanded state and a contracted state by relative movement of the operating lever; a hollow jointing tube having a front end and a rear end, wherein the front end of the hollow jointing tube and the fixing end of the expanding device form a detachable connection; an auxiliary expanding device having a jointing end and a manipulating end, wherein the jointing end of the auxiliary expanding device and the rear end of the hollow jointing tube form a detachable connection; and
an injection tool adapted for connection to the rear end of the hollow jointing tube or to the manipulating end of the operating lever, and the injection tool is adapted to inject the medical filler into the three-dimensional porous device via the hollow jointing tube or via the operating lever; wherein the auxiliary expanding device is configured to expand the expanding device by way of the operating lever, the auxiliary expanding device is configured to be detachable from the expanded expanding device, and the injection tool is configured to be installable with the expanded expanding device in order to fill the medical filler.

The above hollow jointing tube (for example, as shown in FIG. 1a, with numeral 400) may be any conventional hollow jointing tube.

The above-mentioned auxiliary expanding device (see, for example, reference numeral 500 in FIG. 1a) is used to adjust the expanding device to an expanded state or a contracted state by using its connection with the operating lever and the connection between the operating lever and the expanding device. After expanding the expanding device, the detachable connection between the auxiliary expanding device and the hollow jointing tube is used to disassemble the auxiliary expanding device, and then the injection tool is installed to inject the medical filler.

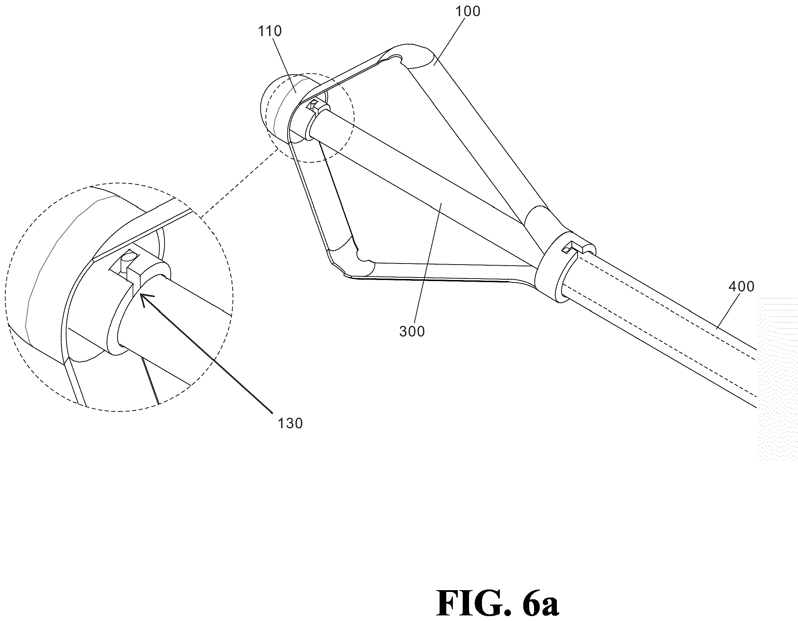

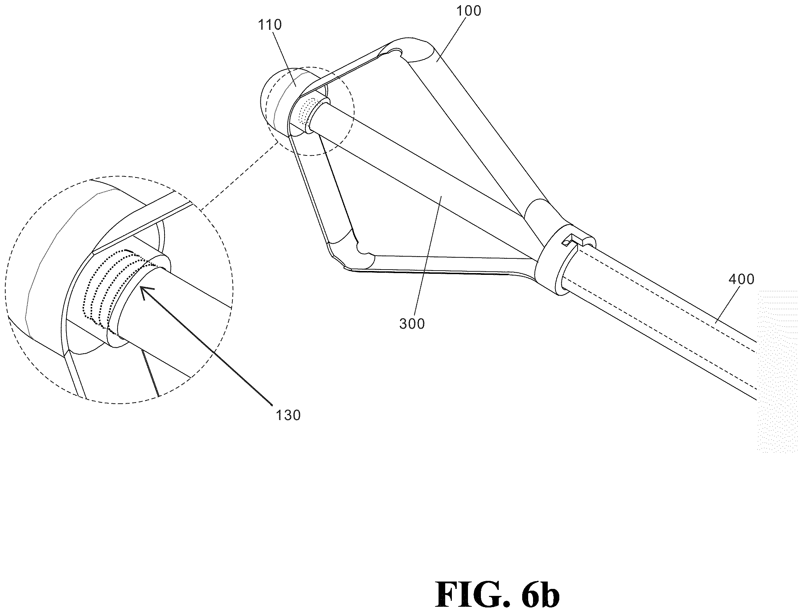

The above-mentioned detachable connection method can be any conventional detachable connection method (similar to those shown in FIGs. 6a and 6b), such as snapping, locking, buckling, screwing, etc.

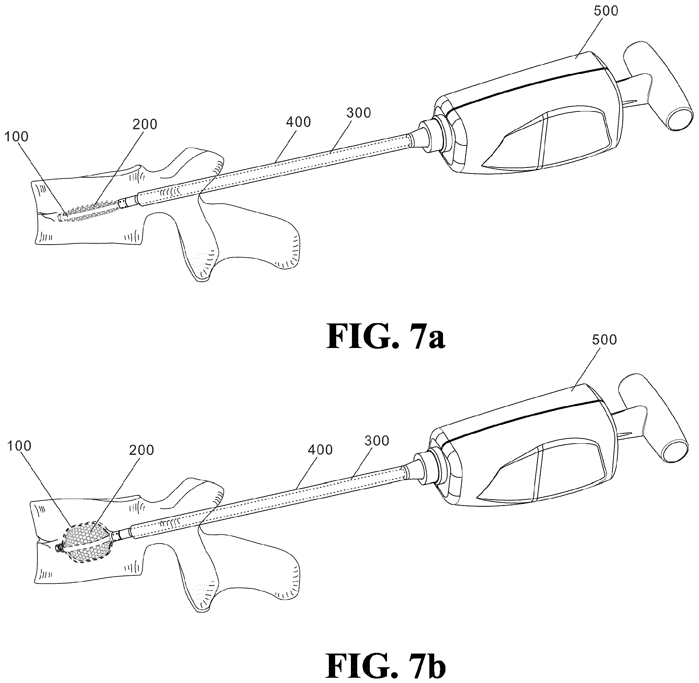

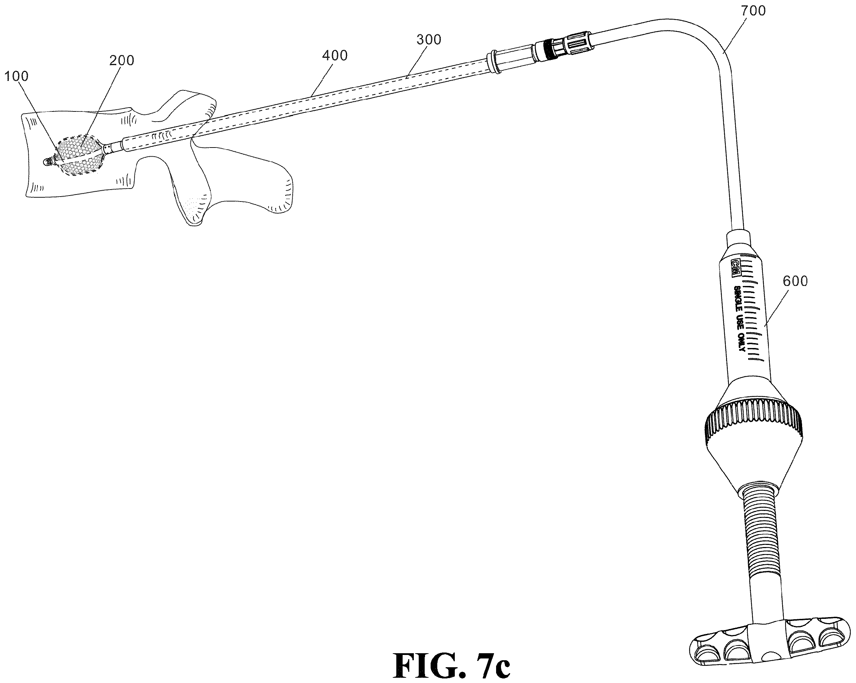

The operating lever mentioned above may be used to adjust the expanding device to be in the expanded state or the contracted state by the relative movement of stretching or rotating with the expanding device (as shown in FIGs. 4a-4d, 7b, 7c).

The operating lever mentioned above may further be a hollow operating lever, and the hollow operating lever inside the expanding device has one or more perforations allowing the medical filler to flow to the porous device through the perforations. The perforation can be any form of perforation, such as grooves, pores, etc.

The operating lever may further include a second detachable mechanism (for example, as shown in FIGs. 1a, 1b, 1c, with numeral 330). After the medical filler is filled in the expanding device and the porous device, the second detachable mechanism detaches the expanding device from at least part of the operating lever, and leaves the expanding device and the porous device in the vertebrae. The second detachable mechanism may be any conventional detachable mechanism, such as screwing, engaging, locking or buckling, while screwing is preferred.

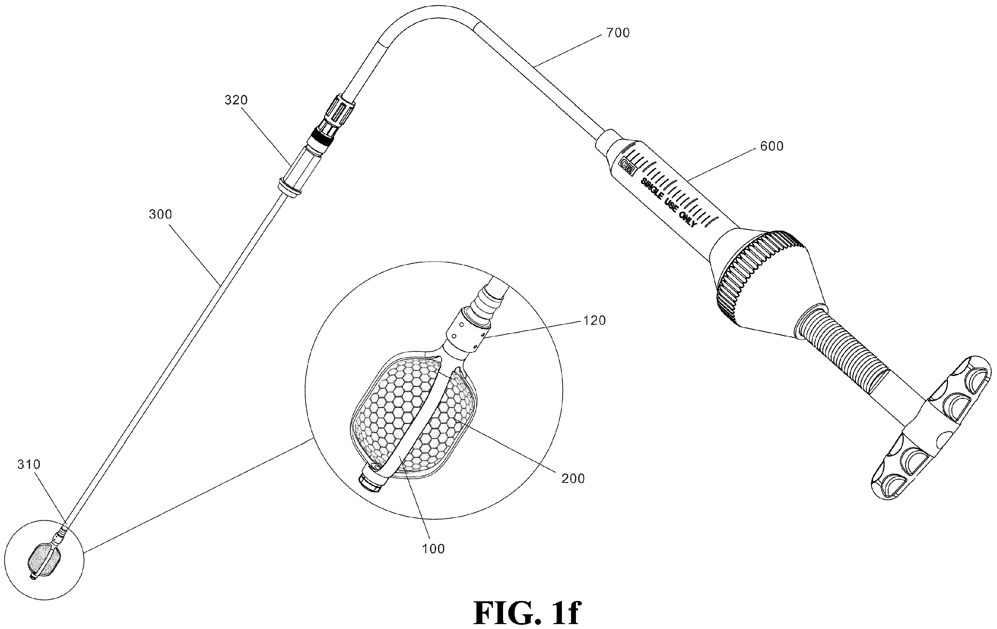

For the above injection tool (as shown in FIG. 1f, numeral 600), the injection method may include: detaching the auxiliary expanding device and the operating lever before infusion, and then connecting the injection tool with the hollow jointing tube for infusion. The injection method also may include: detaching the auxiliary expanding device before the infusion, and then connecting the injection tool with the hollow operating lever for infusion.

The above vertebral fixation device may further include an extension tube (see FIG. 1f, reference numeral 700). The extension tube is used together with the injection tool, which is respectively connected with the rear end of the hollow jointing tube or the manipulating end of the operating lever, and connected with the injection tool; the connection can be any conventional connection method, such as snapping, locking, screwing, etc.

The extension tube may be any conventional extension tube.

The injection tool can be any conventional injection tool.

The vertebral fixation device above may further include a blocking device used to connect the fixing end of the expanding device after perfusing or inserting the medical filler completely, and the medical filler is blocked by the blocking device from flowing out or dropping out. The connection between the blocking device and the fixing end of the expanding device can be any conventional connection, such as screwing, snapping, locking, buckling, etc., the connection method therein is preferred to be screwing or engaging.

### BRIEF DESCRIPTION OF THE DRAWINGS

The present invention will be apparent to those skilled in the art by reading the following detailed description of a preferred embodiment thereof, with reference to the attached drawings, in which:
FIG. 1a is a schematic diagram of a vertebral fixation device according to a preferred specific embodiment of the present invention.
FIG. 1b is a schematic diagram of an expanding device and a porous device of a vertebral fixation device in an expanded state according to the present invention.
FIG. 1c is an enlarged cross-sectional view of an expanding device and a porous device of a vertebral fixation device according to the present invention.
FIG. 1d is an enlarged cross-sectional view of an expanding device, a porous device, and a operating lever of a vertebral fixation device according to the present invention.
FIG. 1e is another schematic diagram of the porous device and the operating lever of FIG. 1d.
FIG. 1f is a schematic diagram of a vertebral fixation device according to a preferred embodiment of the present invention.
FIG. 1g is a schematic diagram of a vertebral fixation device according to another preferred embodiment of the present invention.
FIG. 1h is a schematic diagram of a vertebral fixation device according to another preferred embodiment of the present invention.
FIG. 1i is a schematic diagram of an expanding device and a porous device in the expanded state of the vertebral fixation device according to another preferred embodiment of the present invention.
FIGs. 2a-2o are schematic diagrams of the expanding devices of a vertebral fixation device according to preferred specific embodiments of the present invention.
FIGs. 3a-3c are schematic diagrams of three porous devices of the vertebral fixation device according to preferred specific embodiments of the present invention.
FIGs. 4a-4d are schematic diagrams of two expanding methods of a vertebral fixation device (methods are not claimed).
FIGs. 5a, 5b are respectively schematic diagrams of the connection relationship between two expanding devices and operating lever according to preferred specific embodiments of the present invention.
FIGs. 6a, 6b are respectively schematic diagrams of the connection relationship between tops of the two expanding devices of a vertebral fixation device and an operating lever according to preferred specific embodiments of the present invention.
FIGs. 7a-7d are schematic diagrams of the surgical steps of a vertebral fixation device according to the present invention.

### DETAILED DESCRIPTION OF THE PREFERRED EMBODIMENT

The following description with drawings and element symbols provides more detail of the embodiment of the present invention, so that those skilled in the art can implement the present invention after studying this specification.

FIG. 1a is a schematic diagram of a vertebral fixation device according to a preferred specific embodiment of the present invention. A porous device 200 is arranged inside an expanding device 100, in which a front end 210 of the porous device and a connecting end 220 of the porous device respectively use a ring-shaped fixing member 250 (not shown in FIG. 1a, refer to FIGs. 1c and 1e) to connect with a top end 110 of the expanding device and a fixing end 120 of the expanding device, so as to prevent deviation of the porous device 200 due to the excessive infusion pressure of the medical filler during infusion. When the expanding device 100 is in a contracted state, the porous device 200 is also in a compressed state. The operating lever 300 has a second detachable mechanism 330. After the medical filler is filled into the expanding device 100 and the porous device 200 via the operating lever 300, the expanding device 100 and at least part of the operating lever 300 is detached by way of the second detachable mechanism 330, and the expanding device 100, the porous device 200, and the medical filler are left in the vertebrae. An operating end 320 of the operating lever is connected to a jointing end 510 of the auxiliary expanding device 500, and a manipulating end 520 of the auxiliary expanding device carries the operating lever 300 to control the expanding device 100 to expand and contract.

FIG. 1b is a schematic diagram of the expanding device 100 and the porous device 200 of FIG. 1a in an expanded state. After the expanding device 100 is expanded, the internal porous device 200 automatically expands and fills the space inside the expanding device 100.

FIG. 1c is an enlarged cross-sectional view of the expanding device 100 and the porous device 200 of FIG. 1b. After the expanding device 100 is expanded to be in the expanded state, the expanding device 100 is maintained in the expanded state by the engaging and fixing mechanism 340 of the operating lever 300, so as to facilitate the subsequent expansion of the porous device 200 which is to be filled to the expanding device 100. After the medical filler is injected into the porous device 200 and the expanding device 100, the second detachable mechanism 330 can be used to detach the expanding device 100 and at least part of the operating lever 300, and leave the expanding device 100 and the porous device 200 in the vertebrae.

FIG. 1d is an enlarged cross-sectional view of the expanding device 100, the porous device 200, and the operating lever 300 of FIG. 1a. The porous device 200 is used to fix the front end 210 and the connecting end 220 respectively to the top end 110 and the fixing end 120 of the expanding device through the ring-shaped fixing member 250.

FIG. 1e is another schematic diagram of the porous device 200 and the operating lever 300 of FIG. 1d.

FIG. 1f is a schematic diagram of a vertebral fixation device according to a preferred embodiment of the present invention. After expanding and detaching the hollow jointing tube 400 and the auxiliary expanding device 500 (as shown in FIGs. 1a and 1b) by the expanding device 100, the manipulating end 320 of the operating lever is connected to an extension tube 700, and the connection between the manipulating end 320 of the operating lever and the extension tube 700 is a detachable connection. The medical filler (not shown in the FIG.) is injected into the porous device through the injection tool 600, the extension tube 700, and the operating lever 300. After the medical filler (not shown) is injected to the porous device 200 by the injection tool 600, the extension tube 700 can be detached through a detachable connection, and the second detachable mechanism 330 (refer to FIGs. 1a and 1b) can be used to detach the expanding device 100 and at least part of the operating lever 300, and leave the expanding device 100 and the porous device 200 in vertebrae.

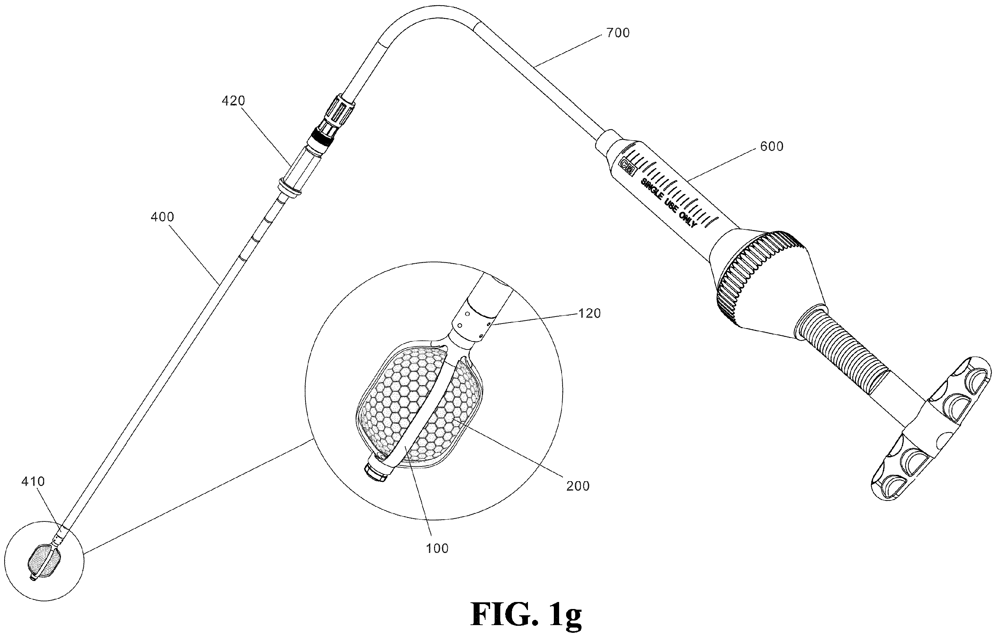

FIG. 1g is a schematic diagram of a vertebral fixation device according to another preferred embodiment of the present invention. A front end 410 of the hollow jointing tube is connected to the expanding device 100 by a detachable connection, and after the expanding device 100 is expanded and the operating lever 300 and the auxiliary expanding device 500 are detached (refer to FIGs. 1a and 1b), connection between a rear end 420 of the hollow jointing tube and the extension tube 700 is also a detachable connection. After the medical filler is injected into the porous device 200 by the injection tool 600, the extension tube 700 and the hollow jointing tube 400 can be detached through the detachable connection of the rear end 420 of the hollow jointing tube. Also, the detachable connection (refer to FIGs. 1a and 1b) of the front end 410 of the hollow jointing tube can be used to detach the expanding device 100 and the hollow jointing tube 400, and leave the expanding device 100 and the porous device 200 in vertebrae.

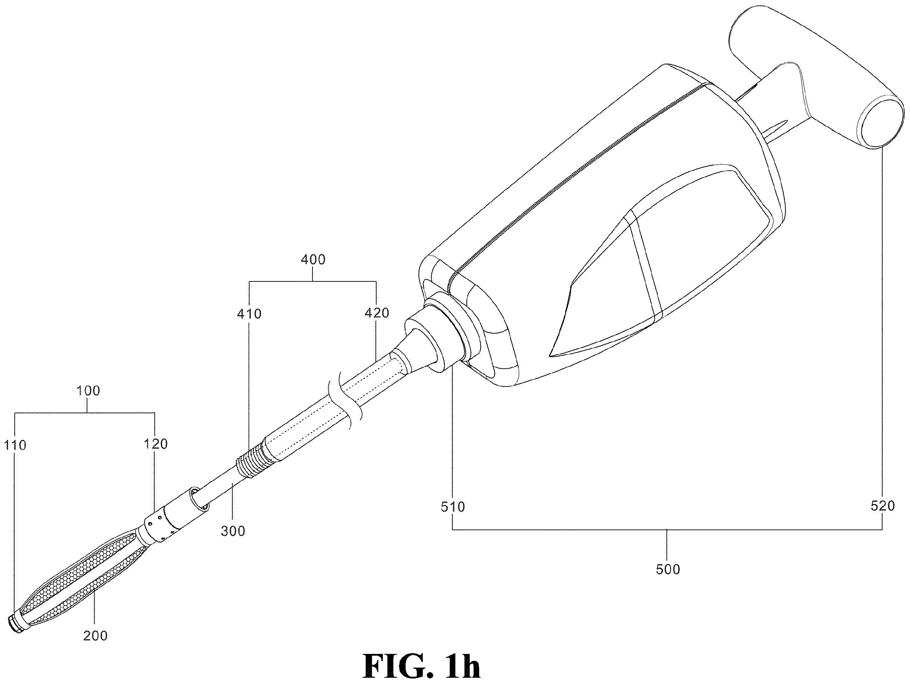

FIG. 1h is a schematic diagram of a vertebral fixation device according to another preferred embodiment of the present invention. The porous device 200 is arranged inside the expanding device 100, in which the front end 210 of the porous device and the connecting end 220 of the porous device respectively use the ring-shaped fixing member 250 (not shown in FIG. 1h, refer to FIGs. 1d and 1e) to connect with the top end 110 of the expanding device and the fixing end 120 of the expanding device, so as to prevent deviation of the porous device 200 due to the excessive infusion pressure of the medical filler during infusion. When the expanding device 100 is in the contracted state, the porous device 200 is also in a compressed state. The fixing end 120 of the expanding device and the jointing end 310 of the operating lever are connected by a screwed first detachable mechanism 130 (as shown in FIG. 6b). After the medical filler is injected into the porous device 200, the expanding device 100 is detached from the operating lever 300 by way of the first detachable mechanism 130, and the expanding device 100 is left in the vertebrae with the porous device 200 and the medical filler therein. The operating end 320 of the operating lever and a jointing end 510 of an auxiliary expanding device 500 are connected. A manipulating end 520 of the auxiliary expanding device is used to drive the operating lever 300 to control the expansion and contraction of the expanding device 100.

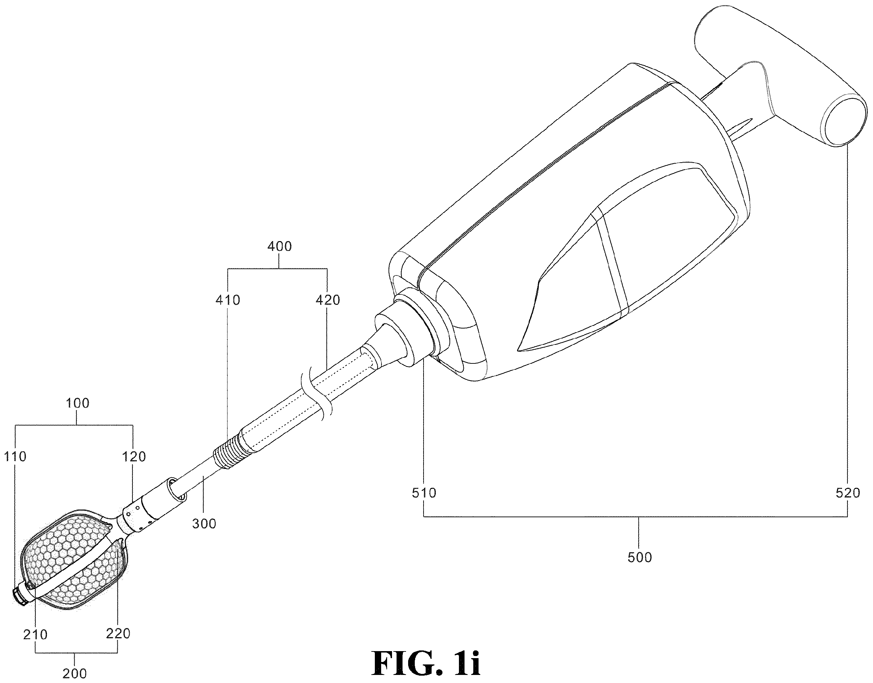

FIG. 1i is a schematic diagram of the expanding device 100 and the porous device 200 of FIG. 1h in the expanded state. After the expanding device 100 is expanded, the internal porous device 200 automatically expands and fills the space inside the expanding device 100.

FIGs. 2a-2o are schematic diagrams of expanding devices of a vertebral fixation device according to preferred specific embodiments of the present invention. FIGs. 2a, 2c, 2e, and 2g illustrate the expanding device 100 in the contracted state. FIGs. 2b, 2d, 2f, 2h, and 2i illustrate a lantern-shaped expanding device 100 in the expanded state. The hollow operating lever 300 inside the expanding device 100 in FIGs 2b, 2e, and 2f has a perforation 350, and the medical filler may flow from the opening of the fixing end 120 of the expanding device through the perforation 350 of the hollow operating lever 300 into the porous device 200.

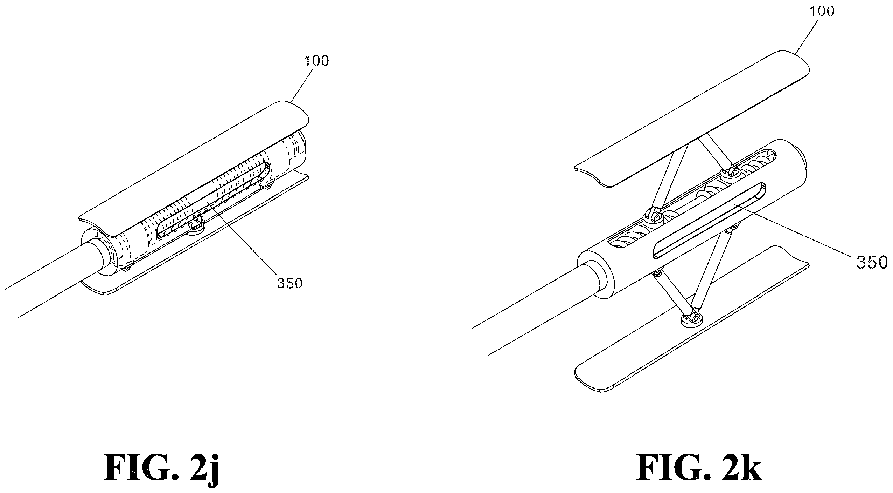

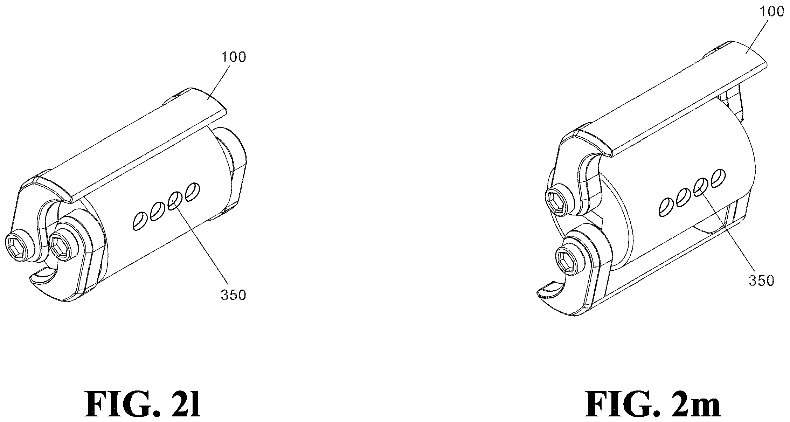

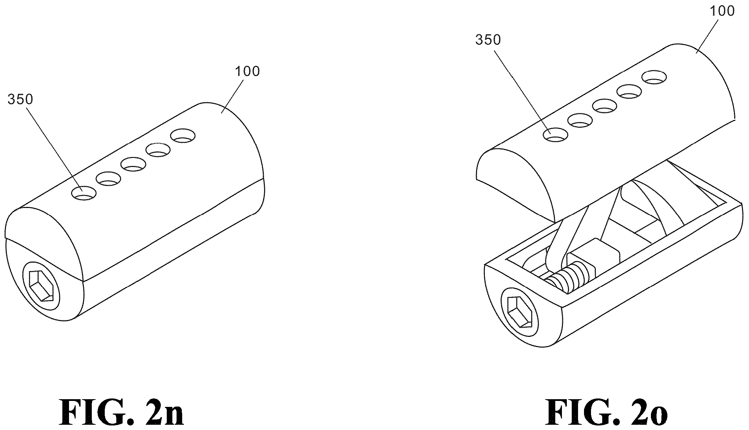

FIG. 2j illustrates a plane-shaped expanding device 100 in the contracted state. FIG. 2k illustrates a plane-shaped expanding device 100 adjusted to be in the expanded state by the operating lever 300. FIG. 2l illustrates a blade-shaped expanding device 100 in the contracted state. FIG. 2m illustrates the blade-shaped expanding device 100 in the expanding state. FIG. 2n illustrates the expanding device 100 in the contracted state. FIG. 2o illustrates the expanding device 100 in the expanded state.

FIGs. 3a-3c are schematic diagrams of three porous devices 200 of the vertebral fixation device according to preferred specific embodiments of the present invention. FIG. 3a is the porous device 200 having a three-dimensional connecting pore structure with pores 230 of uniform size. The size of the pores 230 of the porous device may be controlled to adjust the infusion pressure and the flow smoothness of the medical filler. The material of the structure 240 of the porous device is biodegradable. After the porous device 240 and the medical filler (not shown) are implanted in the human body, the structure 240 of the porous device will slowly degrade in the human body, and multiple connecting channels will be formed in the medical filler. Bone cells can grow into the medical filler through these connecting channels, forming a more intertwined connection with the medical filler.

FIG. 3b illustrates the porous device 200 having a hollow interior and a three-dimensional connecting pore structure with uniformly-sized pores 230 on the periphery. In this way, the medical filler can be injected into the porous device 200 with better fluidity and smoothness, and the flow direction of the medical filler may be controlled through the three-dimensional connecting pore structure on the periphery, and the interdigitate effect of the medical filler may be increased to make closer connection with the vertebrae.

FIG. 3c illustrates the porous device 200 having three-dimensional connecting pore structures with different porosities. The porous device 200 has an the inner layer 260 having a larger porosity and an outer layer 270 having a smaller porosity may be used to increase the smoothness of the medical filler injected to the porous device 200, and reduce the infusion pressure, and the porous device 200 with a smaller porosity in the outer layer 270 is used to effectively achieve interdigitate effect of bone, and to control/limit the flow direction of the medical filler, so as not to cause excessive overflow of the medical filler, which causes danger to the patient.

FIGs. 4a-4d are schematic diagrams of two expanding methods of a vertebral fixation (methods are not claimed). FIGs. 4a-4d illustrate the expanding device 100 extended by the relative stretching motion of the operating lever 300 and the expanding device 100 from the contracted state to the expanded state.

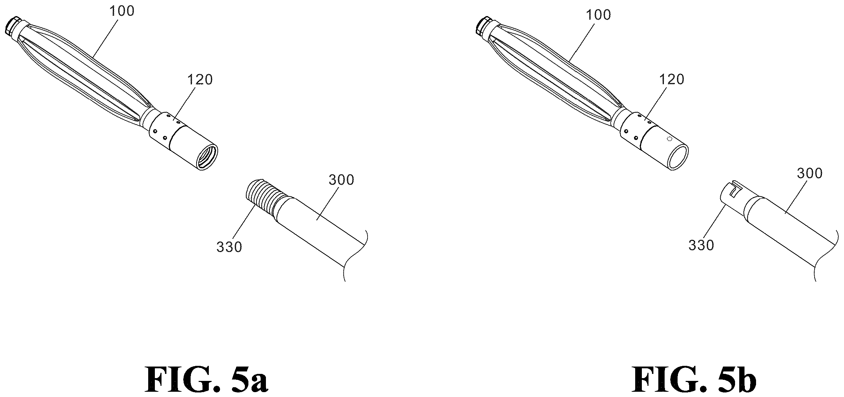

FIGs. 5a, 5b are respectively schematic diagrams of the connection relationship between two expanding devices 100 and operating lever 300 according to preferred specific embodiments of the present invention. FIG. 5a illustrates that the operating lever 300 is connected to the expanding device 100 by the screwed second detachable mechanism 330, so as to inject the medical filler, and then the expanding device 200 is detached from the operating lever 300 or part of the operating lever 300, and the expanding device 100 is left in the vertebrae. FIG. 5b illustrates that the operating lever 300 can be connected to the expanding device 100 by the engaged second detachable mechanism 330.

FIGs. 6a, 6b are schematic diagrams of preferred specific examples of the connection relationship between the tip 110 and the operating lever 300 of the two kinds of expanding devices of the vertebral fixation device of the present invention. FIG. 6a illustrates the top end 110 of the expanding device and the operating lever 300 forming a detachable connection by engaging. FIG. 6b illustrates the top end 110 of the expanding device and the operating lever 300 forming a detachable connection by screwing.

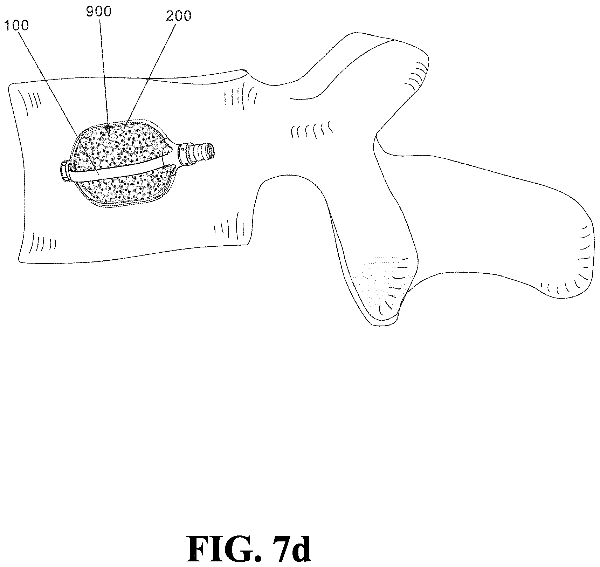

FIGs. 7a-7d are schematic diagrams of surgical steps of a vertebral fixation device according to the present invention. FIG. 7a illustrates the expanding device 100 and the porous device 200, in the contracted state, are placed in the vertebrae. FIG. 7b illustrates that the operating lever 300 and the auxiliary expanding device 500 are used to expand the expanding device 100 from the contracted state to the expanded state and expand a space in the vertebrae, the porous device 200 inside the expanding device 100 will expand and fill the space inside the expanding device 100. FIG. 7c illustrates that the auxiliary expanding device 500 is detached, and the injection tool 600 and the extension tube 700 are connected, and the medical filler (not shown, refer to FIG. 7d) is injected into the porous device 200 and the expanding device 100 through the extension tube 700 and the operating lever 300 through the injection tool 600. FIG. 7d illustrates that the injection tool 600 and the extension tube 700 are detached, and the expanding device 100 is detached from part of the operating lever 300 by the second detachable mechanism 330 of the operating lever 300, and the expanding device 100 and the porous device 200 are left in the vertebrae.

The above description is only used to explain the preferred embodiments of the present invention, and is not intended to limit the present invention in any form.

## Claims

1. A vertebral fixation device comprising:
an expanding device (100) having a fixing end (120) and a top end (110), wherein the expanding device (100) is configured to be adjusted between an expanded state and a contracted state; and
a three-dimensional porous device (200) having a front end (210) and a connecting end (220), wherein the front end (210) of the three-dimensional porous device (200) is connected with the top end (110) of the expanding device (100), and the connecting end (220) of the three-dimensional porous device (200) is adapted for fixing to the fixing end (120) of the expanding device (100);
**characterized in that** the three-dimensional porous device (200) is disposed inside the expanding device (100),
and **in that** the expanding device (100) and the three-dimensional porous device (200) are configured to be placed into the vertebrae, the expanding device (100) is configured to be adjusted to an expanded state to expand a space, and the three-dimensional porous device (200) inside the expanding device (100) is configured to expand and fill into the space, a medical filler (900) is fillable into the three-dimensional porous device (200) from the fixing end (120) of the expanding device (100),
and **in that** the three-dimensional porous device (200) is configured effectively to control a flow direction and infusion range of the medical filler.

2. The vertebral fixation device of claim 1, **characterized in that** the vertebral fixation device further comprises an operating lever (300) having a connecting end (310) and a manipulating end (320); wherein the connecting end (310) is configured to be connected to the top end (110) of the expanding device (100), and wherein the expanding device (100) is configured to be adjusted to the expanded state or contracted state by relative movement with the operating lever (300).

3. The vertebral fixation device of claim 2, **characterized in that** the operating lever (300) is a hollow operating lever (300), and the hollow operating lever (300) inside the expanding device (100) has one or more perforations (350) configured to allow the medical filler (900) to flow into the three-dimensional porous device (200) through the perforations (350).

4. The vertebral fixation device of claim 3, **characterized in that** the expanding device (100) further includes a first detachable mechanism (130), whereupon the expanding device (100) is configured to be detachable from the hollow operating lever (300), and the expanding device (100) is configured to be left in the vertebrae.

5. The vertebral fixation device of claim 3 or 4, **characterized in that** the hollow operating lever (300) has an engaging and fixing mechanism (340), wherein the expanding device (100) is configured to be maintained in the expanded state by the engaging and fixing mechanism (340).

6. The vertebral fixation device of claim 1, **characterized in that** the expanding device (100) is a lantern-shaped expanding device.

7. The vertebral fixation device of claim 1, **characterized in that** the three-dimensional porous device (200) is a three-dimensional connecting pore structure.

8. The vertebral fixation device of claim 1, **characterized in that** the three-dimensional porous device (200) is made of a biodegradable material.

9. The vertebral fixation device of claim 1, **characterized in that** it further comprises:
an operating lever (300) having a connecting end (310) and a manipulating end (320), wherein the connecting end (310) is configured for connection to the top end (110) of the expanding device (100), and wherein the expanding device (100) is configured to be adjusted between the expanded state and the contracted state by relative movement of the operating lever (300);
a hollow jointing tube (400) having a front end (410) and a rear end (420), wherein the front end (410) of the hollow jointing tube (400) and the fixing end (120) of the expanding device (100) form a detachable connection;
an auxiliary expanding device (500) having a jointing end (510) and a manipulating end (520), wherein the jointing end (510) of the auxiliary expanding device (500) and the rear end (420) of the hollow jointing tube (400) form a detachable connection; and
an injection tool (600) adapted for connection to the rear end (420) of the hollow jointing tube (400) or to the manipulating end (320) of the operating lever (300), and the injection tool (600) is adapted to inject the medical filler (900) into the three-dimensional porous device (200) via the hollow jointing tube (400) or via the operating lever (300);
wherein the auxiliary expanding device (500) is configured to expand the expanding device (100) by way of the operating lever (300), the auxiliary expanding device (500) is configured to be detachable from the expanded expanding device, and the injection tool (600) is configured to be installable with the expanded expanding device order to fill the medical filler (900).

10. The vertebral fixation device of claim 9, **characterized in that** the operating lever (300) has a second detachable mechanism, the expanding device (100) is configured to be detachable from the operating lever (300), and the expanding device (100) is configured to be left in the vertebrae.

11. The vertebral fixation device of claim 9 or 10, **characterized in that** the operating lever (300) further includes a fixing mechanism (340), wherein the expanding device (100) is configured to be maintained in the expanded state by the fixing mechanism (340).

## Patentansprüche

1. Wirbelfixierungsvorrichtung umfassend:
eine Spreizvorrichtung (100) aufweisend ein Befestigungsende (120) und ein Oberende (110), wobei die Spreizvorrichtung (100) konfiguriert ist, zwischen einem gespreizten Zustand und einem zusammengezogenen Zustand eingestellt zu werden;
und
eine dreidimensionale poröse Vorrichtung (200) aufweisend ein Vorderende (210) und ein Verbindungsende (220), wobei das Vorderende (210) der dreidimensionalen porösen Vorrichtung (200) mit dem Oberende (110) der Spreizvorrichtung (100) verbunden ist, und das Verbindungsende (220) der dreidimensionalen porösen Vorrichtung (200) eingerichtet ist, an dem Befestigungsende (120) der Spreizvorrichtung (100) befestigt zu werden;
**dadurch gekennzeichnet, dass** die dreidimensionale poröse Vorrichtung (200) innerhalb der Spreizvorrichtung (100) angeordnet ist,
und dass die Spreizvorrichtung (100) und die dreidimensionale poröse Vorrichtung (200) konfiguriert sind, in die Wirbel positioniert zu werden, wobei die Spreizvorrichtung (100) konfiguriert ist, in einen gespreizten Zustand eingestellt zu werden, um einen Raum zu spreizen, und die innerhalb der Spreizvorrichtung (100) befindliche dreidimensionale poröse Vorrichtung (200) konfiguriert ist, sich zu erweitern und den Raum auszufüllen, wobei ein medizinischer Füllstoff (900) in die dreidimensionale poröse Vorrichtung (200) von dem Befestigungsende (120) der Spreizvorrichtung (100) eingefüllt werden kann,
und dass die dreidimensionale poröse Vorrichtung (200) konfiguriert ist, eine Fließrichtung und den Infusionsbereich des medizinischen Füllstoffs wirksam zu kontrollieren.

2. Wirbelfixierungsvorrichtung nach Anspruch 1, **dadurch gekennzeichnet, dass** die Wirbelfixierungsvorrichtung ferner einen Bedienungshebel (300) aufweisend ein Verbindungsende (310) und ein Handhabungsende (320) umfasst, wobei das Verbindungsende (310) konfiguriert ist, mit dem Oberende (110) der Spreizvorrichtung (100) verbunden zu werden, und wobei die Spreizvorrichtung (100) konfiguriert ist, durch eine Relativbewegung mit dem Bedienungshebel (300) in den gespreizten oder den zusammengezogenen Zustand eingestellt zu werden.

3. Wirbelfixierungsvorrichtung nach Anspruch 2, **dadurch gekennzeichnet, dass** der Bedienungshebel (300) ein Hohlbedienungshebel (300) ist, und dass der Hohlbedienungshebel (300), der sich innerhalb der Spreizvorrichtung (100) befindet, eine oder mehrere Perforationen (350) aufweist, die konfiguriert sind, dem medizinischen Füllstoff (900) zu erlauben, durch die Perforationen (350) in die dreidimensionale poröse Vorrichtung (200) zu fließen.

4. Wirbelfixierungsvorrichtung nach Anspruch 3, **dadurch gekennzeichnet, dass** die Spreizvorrichtung (100) ferner einen ersten lösbaren Mechanismus (130) umfasst, woraufhin die Spreizvorrichtung (100) konfiguriert ist, von dem Hohlbedienungshebel (300) gelöst zu werden und die Spreizvorrichtung (100) konfiguriert ist, in den Wirbeln zu verbleiben.

5. Wirbelfixierungsvorrichtung nach Anspruch 3 oder 4, **dadurch gekennzeichnet, dass** der Hohlbedienungshebel (300) einen Eingriff- und Fixiermechanismus (340) umfasst, wobei die Spreizvorrichtung (100) konfiguriert ist, durch den Eingriff-und Fixiermechanismus (340) in dem gespreizten Zustand gehalten zu werden.

6. Wirbelfixierungsvorrichtung nach Anspruch 1, **dadurch gekennzeichnet, dass** die Spreizvorrichtung (100) eine laternenförmige Spreizvorrichtung ist.

7. Wirbelfixierungsvorrichtung nach Anspruch 1, **dadurch gekennzeichnet, dass** die dreidimensionale poröse Vorrichtung (200) eine dreidimensionale Verbindung-Porenstruktur ist.

8. Wirbelfixierungsvorrichtung nach Anspruch 1, **dadurch gekennzeichnet, dass** die dreidimensionale poröse Vorrichtung (200) aus einem biologisch abbaubaren Material besteht.

9. Wirbelfixierungsvorrichtung nach Anspruch 1, **dadurch gekennzeichnet, dass** sie ferner umfasst:
einen Bedienungshebel (300) aufweisend ein Verbindungsende (310) und ein Hanhabungsende (320), wobei das Verbindungsende (310) für die Verbindung mit dem Oberende (110) der Spreizvorrichtung (100) konfiguriert ist, und wobei die Spreizvorrichtung (100) konfiguriert ist, durch eine Relativbewegung des Bedienugshebels (300) zwischen dem gespreizten Zustand und dem zusammengezogenen Zustand eingestellt zu werden;
ein Hohlverbindungsrohr (400) aufweisend ein Vorderende (410) und ein Hinterende (420), wobei das Vorderende (410) des Hohlverbindungsrohr (400) und das Befestigungsende (120) der Spreizvorrichtung (100) eine lösbare Verbindung bilden;
eine Hilfsspreizvorrichtung (500) aufweisend ein Verbindungsende (510) und ein Handhabungsende (520), wobei das Verbindungsende (510) der Hilfsspreizvorrichtung (500) und das Hinterende (420) des Hohlverbindungsrohr (400) eine lösbare Verbindung bilden; und
ein Spritzwerkzeug (600), das für die Verbindung mit dem Hinterende (420) des Hohlverbindungsrohr (400) oder mit dem Handhabungsende (320) des Bedienungshebels (300) eingerichtet ist, und wobei das Spritzwerkzeug (600) eingerichtet ist, den medizinischen Füllstoff (900) durch das Hohlverbindungsrohr (400) oder durch den Bedienungshebel (300) in die dreidimensionale poröse Vorrichtung (200) einzuspritzen;
wobei die Hilfsspreizvorrichtung (500) konfiguriert ist, die Spreizvorrichtung (100) mittels des Bedienungshebels (300) zu spreizen, wobei die Hilfsspreizvorrichtung (500) konfiguriert ist, von der gespreizten Spreizvorrichtung gelöst zu werden, und wobei das Spritzwerkzeug (600) konfiguriert ist, mit der gespreizten Spreizvorrichtung installiert werden zu können, um den medizinischen Füllstoff (900) einzufüllen.

10. Wirbelfixierungsvorrichtung nach Anspruch 9, **dadurch gekennzeichnet, dass** der Bedienungshebel (300) einen zweiten lösbaren Mechanismus umfasst, die Spreizvorrichtung (100) konfiguriert ist, von dem Bedienungshebel (300) gelöst zu werden, und die Spreizvorrichtung (100) konfiguriert ist, in den Wirbeln zu verbleiben.

11. Wirbelfixierungsvorrichtung nach Anspruch 9 oder 10, **dadurch gekennzeichnet, dass** der Bedienungshebel (300) ferner einen Fixiermechanismus (340) umfasst, wobei die Spreizvorrichtung (100) konfiguriert ist, durch den Fixiermechanismus (340) in dem gespreizten Zustand gehalten zu werden.

## Revendications

1. Dispositif de fixation vertébrale, comprenant:
un dispositif d'expansion (100) ayant une extrémité de fixation (120) et une extrémité supérieure (110), ledit dispositif d'expansion (100) étant configuré pour être réglé à un état expansé ou à un état contracté; et
un dispositif poreux tridimensionnel (200) ayant une extrémité avant (210) et une extrémité de connexion (220), l'extrémité avant (210) dudit dispositif poreux tridimensionnel (200) étant connectée à l'extrémité supérieure (110) dudit dispositif d'expansion (100), et l'extrémité de connexion (220) dudit dispositif poreux tridimensionnel (200) étant adaptée pour être fixée à l'extrémité de fixation (120) dudit dispositif d'expansion (100);
**caractérisé en ce que** le dispositif poreux tridimensionnel (200) est disposé à l'intérieur du dispositif d'expansion (100),
et **en ce que** le dispositif d'expansion (100) et le dispositif poreux tridimensionnel (200) sont configurés pour être placés dans les vertèbres, le dispositif d'expansion (100) étant configuré pour être réglé à un état expansé afin d'élargir un espace, et le dispositif poreux tridimensionnel (200) situé à l'intérieur du dispositif d'expansion (100) étant configuré pour s'étendre et remplir l'espace, un produit de remplissage médical (900) pouvant être inséré dans le dispositif poreux tridimensionnel (200) depuis l'extrémité de fixation (120) du dispositif d'expansion (100),
et **en ce que** le dispositif poreux tridimensionnel (200) est configuré pour contrôler efficacement une direction d'écoulement et une plage d'infusion du produit de remplissage médical.

2. Dispositif de fixation vertébrale selon la revendication 1, **caractérisé en ce qu'**il comprend en outre un levier de commande (300) ayant une extrémité de connexion (310) et une extrémité de manipulation (320); dans lequel l'extrémité de connexion (310) est configurée pour être connectée à l'extrémité supérieure (110) du dispositif d'expansion (100), et dans lequel le dispositif d'expansion (100) est configuré pour être réglé à l'état expansé ou contracté par un mouvement relatif du levier de commande (300).

3. Dispositif de fixation vertébrale selon la revendication 2, **caractérisé en ce que** le levier de commande (300) est un levier de commande creux (300), et le levier de commande creux (300) situé à l'intérieur du dispositif d'expansion (100) comporte une ou plusieurs perforations (350) configurées pour permettre au produit de remplissage médical (900) de s'écouler dans le dispositif poreux tridimensionnel (200) à travers les perforations (350).

4. Dispositif de fixation vertébrale selon la revendication 3, **caractérisé en ce que** le dispositif d'expansion (100) comprend en outre un premier mécanisme détachable (130), le dispositif d'expansion (100) étant ainsi configuré pour être détachable du levier de commande creux (300), et le dispositif d'expansion (100) étant configuré pour être laissé dans les vertèbres.

5. Dispositif de fixation vertébrale selon la revendication 3 ou 4, **caractérisé en ce que** le levier de commande creux (300) comporte un mécanisme de fixation et de verrouillage (340), dans lequel le dispositif d'expansion (100) est configuré pour être maintenu dans l'état expansé par le mécanisme de fixation et de verrouillage (340).

6. Dispositif de fixation vertébrale selon la revendication 1, **caractérisé en ce que** le dispositif d'expansion (100) est un dispositif d'expansion en forme de lanterne.

7. Dispositif de fixation vertébrale selon la revendication 1, **caractérisé en ce que** le dispositif poreux tridimensionnel (200) est une structure de pores interconnectés tridimensionnelle.

8. Dispositif de fixation vertébrale selon la revendication 1, **caractérisé en ce que** le dispositif poreux tridimensionnel (200) est fabriqué à partir d'un matériau biodégradable.

9. Dispositif de fixation vertébrale, selon la revendication 1, **caractérisé en ce qu'**il comprend en outre:
un levier de commande (300) ayant une extrémité de connexion (310) et une extrémité de manipulation (320), dans lequel l'extrémité de connexion (310) est configurée pour la connexion à l'extrémité supérieure (110) du dispositif d'expansion (100), et dans lequel le dispositif d'expansion (100) est configuré pour être réglé entre l'état expansé et l'état contracté par un mouvement relatif du levier de commande (300);
un tube de raccordement creux (400) ayant une extrémité avant (410) et une extrémité arrière (420), dans lequel l'extrémité avant (410) du tube de raccordement creux (400) et l'extrémité de fixation (120) du dispositif d'expansion (100) forment une connexion détachable;
un dispositif d'expansion auxiliaire (500) ayant une extrémité de raccordement (510) et une extrémité de manipulation (520), dans lequel l'extrémité de raccordement (510) du dispositif d'expansion auxiliaire (500) et l'extrémité arrière (420) du tube de raccordement creux (400) forment une connexion détachable; et
un outil d'injection (600) adapté pour être connecté à l'extrémité arrière (420) du tube de raccordement creux (400) ou à l'extrémité de manipulation (320) du levier de commande (300), et l'outil d'injection (600) étant adapté pour injecter le produit de remplissage médical (900) dans le dispositif poreux tridimensionnel (200) par le tube de raccordement creux (400) ou par le levier de commande (300);
où le dispositif d'expansion auxiliaire (500) est configuré pour étendre le dispositif d'expansion (100) au moyen du levier de commande (300), le dispositif d'expansion auxiliaire (500) est configuré pour être détaché du dispositif d'expansion étendu, et l'outil d'injection (600) est configuré pour être installé avec le dispositif d'expansion étendu afin d'injecter le produit de remplissage médical (900).

10. Dispositif de fixation vertébrale selon la revendication 9, **caractérisé en ce que** le levier de commande (300) comporte un second mécanisme détachable, le dispositif d'expansion (100) étant configuré pour être détachable du levier de commande (300), et le dispositif d'expansion (100) étant configuré pour être laissé dans les vertèbres.

11. Dispositif de fixation vertébrale selon la revendication 9 ou 10, **caractérisé en ce que** le levier de commande (300) comprend en outre un mécanisme de fixation (340), dans lequel le dispositif d'expansion (100) est configuré pour être maintenu dans l'état expansé par le mécanisme de fixation (340).
